# EUROPEAN PATENT SPECIFICATION

(11) **EP 4 467 085 B1**
(45) Date of publication and mention of the grant of the patent: **08.07.2026**
(21) Application number: 24205559.8
(22) Date of filing: 17.06.2021
(51) Int. Cl.: A61B 17/42, A61B 17/12

(54) **BALLOON OCCLUDER**
BALLONOKKLUDER
DISPOSITIF D'OCCLUSION À BALLONNET

(30) Priority: 18.06.2020 US 202063040824 P
(43) Date of publication of application: 27.11.2024
(62) Divisional of application: 21746592.1
(73) Proprietor: CONMED Corporation, Largo, FL 33773 (US)
(72) Inventor: VENDERLEY, Melanie, Englewood, CO 80113 (US); WILLIAMS, Mason, Centennial, CO 80122 (US)
(74) Representative: Strehl & Partner mbB

(56) References cited:
- EP-A1- 3 461 443
- EP-A1- 3 556 306
- US-A- 2 769 442
- US-A- 5 104 377
- US-A1- 2007 078 476
- US-A1- 2010 168 784
- US-A1- 2014 358 158
- US-A1- 2019 254 708

## Description

### Cross Reference to Related Application

The present application claims priority to and the benefit of U.S. Provisional Patent Application Number 63/040,824, filed June 18, 2020.

### Field of the Invention

The present disclosure relates generally to devices and methods for manipulation of the uterus and cervix in surgical and diagnostic procedures.

### Background

Various conventional forms of uterine manipulators with occluders are known. For example, U.S. Pat. Application No. 15/617,299, assigned to Applicant, describes a uterine manipulator generally consisting of an elongated cannulated tube comprising a proximal end and a distal end (to the medical practitioner using the device); a cervical cup having a top proximal portion of a first diameter and a base distal portion of a second smaller diameter, wherein: the base distal portion includes a hole formed therein having a perimeter including a distal end and a proximal end, the elongated cannulated tube is positioned through the hole in the cervical cup; and a plastic or a foam occluder is positioned proximally to the cervical cup to maintain the pneumoperitoneum for the procedure (see also U.S. Pat. No. 10695092, assigned to Applicant).

The uterine manipulators with occluders are used to maneuver and visualize the uterus during various medical examinations and laparoscopic procedures while maintaining pneumoperitoneum. Such examinations and procedures include a complete, total laparoscopic hysterectomy, a partial laparoscopic hysterectomy, and a colpotomy. Specifically, the occluder can form a seal to prevent loss of pneumoperitoneum during colpotomy and displaces the sigmoid colon away from the uterus. Other conventional forms of uterine manipulators with occluders exist and contain similar features.

However, conventional uterine manipulators with occluders do not include a mechanism to allow the occluder be removed or used independently of the uterine manipulator. Indeed, conventional occluders are not able to be used during both pre-colpotomy and post-colpotomy. Conventional occluders such as those made from foam or plastic can degrade over time. Furthermore, conventional devices are typically one size and do not allow proper conformity to a patient's anatomy or manipulation. Additionally, some conventional uterine manipulators and occluders do not include a mechanism or structural configuration to reduce hand fatigue during use. Accordingly, there is a need in the art for improved devices and methods for manipulation and maintenance of the uterus in surgical and diagnostic procedures including a mechanism or structural configuration to address each of these and other shortcomings of conventional devices.

Description of the Related Art Section Disclaimer: To the extent that specific patents/publications/products are discussed above in this Background Section or elsewhere in this Application, these discussions should not be taken as an admission that the discussed patents/publications/products are prior art for patent law purposes. For example, some or all of the discussed patents/publications/products may not be sufficiently early in time, may not reflect subject matter developed early enough in time and/or may not be sufficiently enabling so as to amount to prior art for patent law purposes.

US 2010/168784 A1 discloses a balloon catheter assembly with the features in the preamble of present claim 1. Other conventional devices in this field are described in US 2007/078476 A1, US 2 769 442 A, EP 3 556 306 A1, US 2014/358158 A1, EP 3 461 443 A1, US 5 104 377 A, and US 2019/254708 A1.

### Summary of the Invention

The present disclosure is directed to inventive devices and methods for manipulation of the uterus and vaginal occlusion in surgical and diagnostic procedures, which overcome the various problems with conventional devices (as discussed herein and below). A particular non-limiting goal of utilization of the embodiments and implementations herein is to provide a device for manipulation of the uterus and injection of fluids or gases during laparoscopic procedures such as laparoscopic assisted vaginal hysterectomy (LAVH), total laparoscopic hysterectomy (TLH), minilap, laparoscopic tubal occlusion or diagnostic laparoscopy (and other similar procedures as should be understood by a person of ordinary skill in the art in conjunction with a review of this disclosure), and for the maintenance of a pneumoperitoneum by sealing the vagina during such procedures. In brief, the uterine manipulator device of an embodiment allows a medical practitioner to more easily conform and manipulate the uterus and seal the vagina during procedures by using a balloon occluder. Specifically, to maintain pneumoperitoneum during use, the occluder contains an inflatable balloon for vaginal occlusion by conforming to patient anatomy.

The balloon occluder can be structured and/or configured to be removed or used independently of the uterine manipulator allowing for vaginal occlusion during both pre-colpotomy and post-colpotomy. The balloon occluder is configured to be inflated and deflated. Following colpotomy and subsequent removal of the device, the balloon occluder component can be removed from the uterine manipulator. This independent balloon occluder component can then be reinserted into the vaginal canal and inflated acting as a standalone occluder during suturing of the vaginal cuff following hysterectomy. Applicant has recognized and appreciated that it would be beneficial for medical practitioners to be able to approach such procedures with a higher degree of confidence in performing a consistent, predictable and repeatable procedure.

Additionally, the device can be configured to reduce hand fatigue during use due to the improved ergonomics of the device handle and the presence of a low-profile silicone cap. The scope of protection is defined in the appended claims.

Generally, a uterine manipulator device includes an elongated cannulated tube comprising a proximal end and a distal end; a handle positioned on the proximal end of the elongated cannulated tube; a cervical cup positioned on the elongated cannulated tube; an intrauterine balloon positioned on the distal end of the elongated cannulated tube; a stem positioned proximally from the cervical cup on the elongated cannulated tube; and a balloon occluder assembly including a balloon occluder shaped to form an opening, the opening configured to allow passage therethrough of the elongated cannulated tube and the cervical cup.

In one aspect, the balloon occluder assembly further comprises an inflation valve communicatively coupled to the balloon occluder via a gas passage lumen.

In a further aspect, the balloon occluder is torus shaped.

In a further aspect, a square key feature is in the handle which directs an applied force in one direction only.

In a further aspect, the stem comprises a locking assembly positioned proximally from the distal lip of the stem, wherein the locking assembly is configured to lock the stem and the balloon occluder assembly from movement in at least one direction along the elongated cannulated tube.

In a further aspect, the stem comprises a plurality of ribs positioned between a proximal lip and distal lip configured to limit movement of the balloon occluder assembly.

In a further aspect, the opening of the balloon occluder is configured to allow passage therethrough of the distal lip of the stem.

In a further aspect, the balloon occluder is communicatively coupled to a pilot balloon configured to indicate balloon inflation.

Generally, in another aspect a uterine manipulator device comprises an elongated cannulated tube comprising a proximal end and a distal end, a stem positioned along the elongated cannulated tube, the stem comprising a plurality of ribs positioned between a proximal lip and distal lip; and a balloon occluder assembly, comprising: a balloon shaped to form an opening, the opening configured to allow passage therethrough of the elongated cannulated tube and the distal lip of stem; and an inflation valve configured to change the balloon occluder between an inflated stated and a deflated stated, inflation valve being communicatively coupled to the balloon occluder via a gas passage lumen.

In a further aspect, the stem comprising a locking assembly positioned proximally from the distal lip, wherein the locking assembly is configured to lock the stem and the balloon occluder assembly from movement in at least one direction along the elongated cannulated tube.

In a further aspect, a handle is positioned on the proximal end of the elongated cannulated tube.

In a further aspect, the balloon occluder assembly further comprises a pilot balloon configured to indicate balloon inflation.

In a further aspect, the balloon occluder assembly is configured to be placed around the ribs of the stem.

In an embodiment of the invention as defined in appended claim 1, a balloon occluder comprises a torus shaped balloon configured to be changed from a deflated state to an inflated state and comprising an inner wall and an outer wall; and an inflation valve communicatively coupled to the balloon via a gas passage lumen. The balloon occluder is constructed from a single silicone molded component, which is folded over on itself and sealed circumferentially, so that the seal separates a first axial portion with only a single layer of material from a second axial portion with two layers which form a pocket for air.

In a further aspect, the device comprising a pilot balloon configured to indicate balloon inflation.

In a further aspect, the outer wall of balloon further comprising a lumen port.

### Brief Description of the Drawings

The present invention will be more fully understood and appreciated by reading the following Detailed Description in conjunction with the accompanying drawings. The accompanying drawings illustrate only typical embodiments of the disclosed subject matter and are therefore not to be considered limiting of its scope, for the disclosed subject matter may admit to other equally effective embodiments.

Reference is now made briefly to the accompanying drawings, in which:
FIG. 1 is a side perspective view of a uterine manipulator with balloon occluder device;
FIG. 2A is a side perspective view of a balloon occluder assembly;
FIG. 2B is a side perspective view of a balloon occluder assembly;
FIG. 3A is a side perspective view of a balloon occluder assembly;
FIG. 3B is a side perspective view of a balloon occluder assembly;
FIG. 4 is a perspective view of a stem;
FIG. 5A is a top schematic sectional view of a balloon occluder along G-G of FIG. 5B according to an embodiment;
FIG. 5B is a side schematic view of a balloon occluder according to an embodiment;
FIG. 5C is a bottom schematic sectional view of a balloon occluder along D-D of FIG. 5B according to an embodiment;

Where applicable, like reference characters designate identical or corresponding components and units throughout the several views, which are not to scale unless otherwise indicated. Moreover, the embodiments disclosed herein may include elements that appear in one or more of the several views or in combinations of the several views.

### Detailed Description of Embodiments

Aspects of the present invention and certain features, advantages, and details thereof, are explained more fully below with reference to the non-limiting examples illustrated in the accompanying drawings. Descriptions of well-known structures are omitted so as not to unnecessarily obscure the invention in detail. It should be understood, however, that the detailed description and the specific non-limiting examples, while indicating aspects of the invention, are given by way of illustration only, and are not by way of limitation.

As used herein for purposes of the present disclosure, the terms "distal" and "proximal" are used to describe locations of embodiments of the device from the perspective of a medical practitioner using the device.

The uterine manipulator with balloon occluder device (referred to generally by reference numeral 100) can include a uterine manipulator (referred to generally by reference numeral 1) and a balloon occluder assembly (referred to generally by reference numeral 20). The uterine manipulator with balloon occluder device 100 can comprise a cannulated manipulator tube having a proximal end 4 and a distal end 6, a handle 11 extending from the proximal end 4, and a balloon occluder assembly 20 positioned distally from the handle 11 along the cannulated manipulator tube 5.

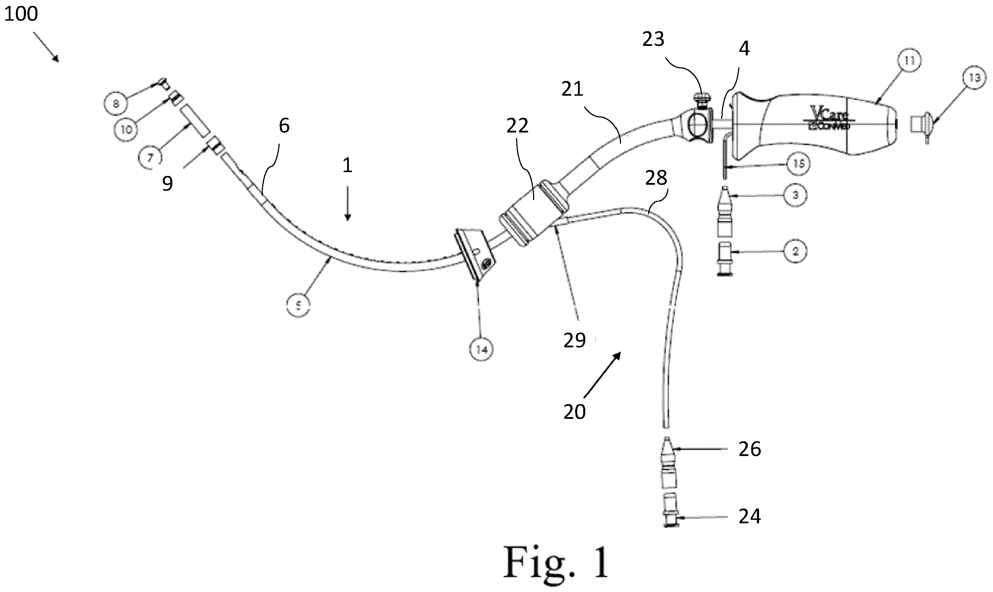

Referring to FIG. 1, there is shown a perspective view of a uterine manipulator with balloon occluder device 100. As shown, the uterine manipulator with balloon occluder device 100 comprises a uterine manipulator 1 having a handle 11 with a dye injection port cap 13 (positioned preferably through the proximal end of handle 11), a cannulated manipulator tube 5 extending from the handle, a balloon occluder assembly 20 moveably positioned along the proximal portion of cannulated manipulator tube 5, and a cervical cap 14 positioned along the tube 5 adjacent to the balloon occluder assembly 20. Extending from the distal end of the cannulated manipulator tube 5 is a proximal heat shrink 9, an intrauterine balloon 7, a distal heat shrink 10, and a cap 8. Additionally, attached to the handle 11 is a lumen 15, a pilot balloon 3, and a check valve 2 all communicatively coupled to the intrauterine balloon 7. The balloon occluder assembly 20 can comprise balloon occluder 22 communicatively attached to check valve 24 and pilot balloon 26 via lumen 28.

The handle 11, which can be smooth (as shown) and conform to a user's hand as a whole or provide for the positioning of all four fingers on one side and the thumb on the opposite side (which can include a gripping/non-smooth surface such as a plurality of raised portions or other non-smooth surface structure as should be appreciated by a person of skill in the art in conjunction with a review of this disclosure), allows for easy manipulation of the uterus up, down and sideways. The dye injection port cap 13 can be positioned on the handle at multiple locations but preferably on the proximal portion of handle 11 for example as shown.

The cannulated manipulator tube 5 can be s-curved or curved at its proximal end 4 and straight at its distal end 6 for easy introduction of the device 10, for manipulation of both retroverted and anteverted uteri, and for maintaining proper attitude of the uterus. The manipulator tube 5 is configured to anatomically conform to the angle of the sacral curve, and to allow for easy manipulation of the uterus. The cannulated manipulator tube 5 is connected to the handle 11 and the dye injection port cap 13 (preferably on the distal portion of the handle 11) and the pilot balloon 3 through the handle 11. This cannulated manipulator tube 5 is cannulated to allow for injection of fluids via the dye injection port cap 13 for chromopertubation and inflation of balloon 7 through via pilot balloon 3. The dye injection port cap 13 has a low-profile cap which does not extend far from the body of handle 11 when in place. Additionally, cap 13 can be silicone or other similar material.

The manipulator tube 5 can include repeated sets of reference graduations (e.g., 4 cm to 16 cm moving away from either side of the cervical cup 14). The manipulator tube 5 can have any number of sets of markings, for example the manipulator tube 5 can be marked with reference graduations from both the distal end 6 and the proximal end 4. The reference graduations provide a guide for comparison to a graduated uterine sound, and can aid in attaining proper depth of insertion during use. The reference graduations are laser marked on the manipulator tube 5. In conventional devices, reference graduations can be added to the manipulator tube 5 via the process of pad printing. Pad printing can be done prior to application of heat shrink. Although laser marking is typically cheaper than pad printing, it is more accurate and more permanent because, for example, pad printing can be rubbed off. A heat shrink is first applied to the manipulator tube 5 before the reference graduations are laser marked. It has been found and appreciated by the inventors that the acrylated heat shrink allows for an ultraviolet laser to mark the applied heat shrink layer. As such, an ultraviolet laser may be used to successfully mark a thin layer of the acrylated polyolefin on the manipulator tube 5. In such case, the UV laser emits a wavelength of 355 nm. It is contemplated that other wavelengths from the UV laser may be used to successfully create the reference graduations.

Stem 21 is configured to position and hold the balloon occluder assembly 20 in place. Stem 21 is cannulated and is placed around and along manipulator tube 5. The stem 21 can be positioned distally to the handle 11 and proximally to the cervical cup 14. The stem 21 can be flexible enough to be slid along the curves of the manipulator tube 5.

The balloon occluder assembly 20 is moveable along the manipulator tube 5. Stem 21 can be used to position balloon occluder assembly 20. Once in the desired position to block gas from leaving the exposed cavity (as should be understood by a person of ordinary skill in the art in conjunction with a review of this disclosure), the balloon occluder assembly 20 and stem 21 can be locked in place (which also prevents proximal movement of the cervical cup 14). In the example shown, the locking assembly is a thumbscrew 23 in stem 21. With conventional screws, the user does not know how deep the screw is in the collar and thus, when loosening the screw, the screw oftentimes falls out. To permit tightening and loosening of the screw without risking losing the screw, the thumbscrew can be used. The last thread in the thumbscrew 23 can be over-torqued such that there is virtually no pitch between the last two threads. As there is no space between the last two threads, the thumbscrew cannot be entirely removed. Therefore, although the thumbscrew 23 can be loosened and tightened, it cannot be completely removed from, preventing loss of the thumbscrew 23. The screw channel can be composed of or overmolded with hard plastic, polycarbonate, or nylon to permit the thumbscrew 23 to push against it while maintaining the shape of the stem 21 design. Alternatively, other locking mechanisms (e.g., washer, nut, plastic piece, other deformation of the screw) that are bigger than the hole that the screw is positioned through (or otherwise does not allow the screw to come out of the hole) are contemplated.

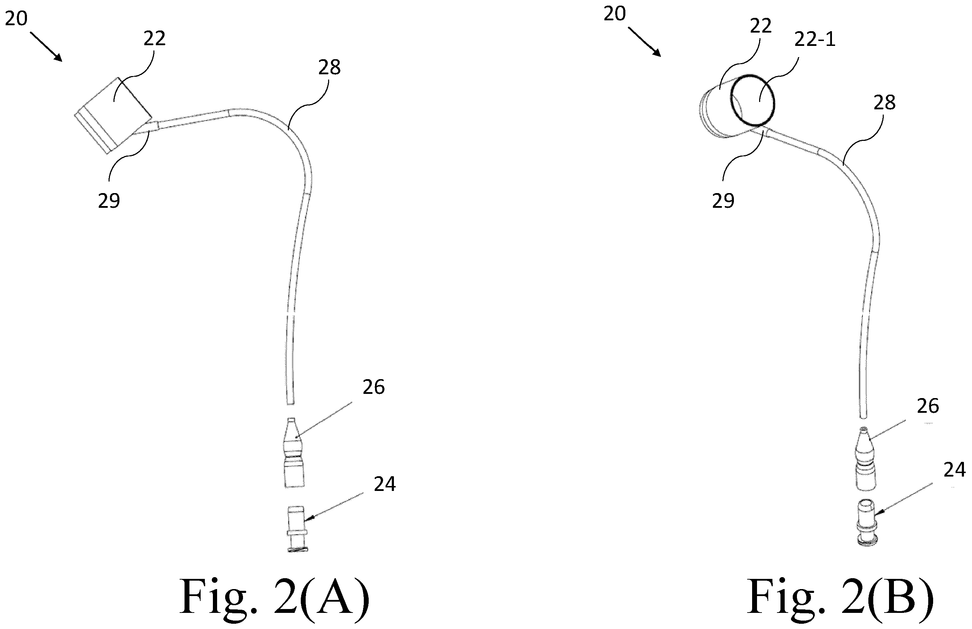

The balloon occluder assembly 20 is shown positioned on the distal portion of stem 21, the stem 21 being positioned through the cannulated lumen 22-1 (or opening) of the balloon occluder 22 (see FIG. 2B). As shown, balloon occluder assembly 20 is configured to allow the manipulator tube 5 to be positioned therethrough balloon occluder 22. The balloon occluder assembly 20 contains an inflatable balloon occluder 22, having an inflated state and an uninflated state, for vaginal occlusion by conforming to patient anatomy. Specifically, the balloon occluder 22 is configured and positioned to keep gas (e.g., insufflation gas) in the exposed cavity to maintain insufflation of the cavity, when in the inflated state. Balloon occluder 22 can be inflated using the check valve 24, pilot balloon 26, and lumen 28 assembly as should be known in the art.

The cervical cup 14 is positioned on the manipulator tube 5 to provide manipulation of the uterus, and retraction and elevation of the cervix. The cervical cup 14 is optionally able to be locked and unlocked in place and prevented from moving proximally on the manipulator tube 5. The cervical cup 14 is positioned on and adjacent to the proximal end 4 of the manipulator tube 5, and can include sites/holes for suturing positioned through the side of the cervical cup 14. The cervical cup 14 can include various volumes and diameters, examples of which are shown in Table 1 below:

**Table 1**

| **Cervical Cup Volume** | **Cervical Cup Diameter** | **Cervical Cup Designation** |
|---|---|---|
| 9.7 cm³(0.59in³) | 32mm (1.26in) | S |
| 14.3cm³(0.87in³) | 34mm (1.34in) | M |
| 20.7cm³(1.26in³) | 37mm (1.46in) | L |
| 26.7cm³(1.63in³) | 40mm (1.57in) | XL |

In the example shown, the cervical cup 14 tapers from a top distal portion with a first diameter to a base proximal portion with a second smaller diameter including a central hole having a perimeter. The perimeter can be chamfered/angled away from the longitudinal axis, and can be straight/not angled with respect to the longitudinal axis (the angling can be reversed). The chamfering of the perimeter aids in the movement of the cervical cup 14 along the manipulator tube 5. The straight/not angled perimeter portion aids in increasing the retention force of the cervical cup 14 on the manipulator tube 5 and in preventing detachment of the cup from the manipulator tube 5. Additionally, the hole diameter can be decreased (from 2.15 cm to 2.05 cm). The combination of the straight/not angled perimeter portion and the narrowing of the diameter of the hole significantly increases the retention force of the cervical cup 14 on the manipulator tube 5. The cervical cup contains a guide for performing colpotomy as well as suture holes for tissue removal.

The intrauterine balloon 7 covers the manipulator tube 5 on its distal portion. The intrauterine balloon 7 is shown positioned on the distal end 6 of the manipulator tube 5. The balloon 7 in conjunction with cap 8 is configured and positioned to reduce the risk of uterine perforation and is used to stabilize the manipulator tube 5 within the uterine cavity. Balloon 7 is configured to be hold up to 45cc of air. This allows better manipulation of larger uteri.

The balloon 7 can be a Thermoplastic Elastomer (TPE) balloon. The TPE balloon is based on a styrene block copolymer, SEBS compound. TPE balloons are similar to PVC balloons in strength and performance, and are composed and configured to maintain inflation of the balloon. Although a TPE balloon 7 is shown and described in conjunction with the uterine manipulator 1, a conventional silicone or urethane-based balloon may be used. However, TPE balloons are less permeable to gas as compared to silicone balloons. As silicone balloons are more permeable to gas, they can deflate. To combat deflation, saline is often used to inflate the silicone balloon as saline will not permeate. TPE balloons achieve similar performance by inflating the balloon with gas. Gas does not strain bonds in the balloon as much as saline, so gas is preferable if inflation can be maintained. However, TPE balloons can be used with saline, at the choice of the user.

In conventional uterine manipulator devices, the balloon is glued in place on both the proximal and distal ends of the balloon (or other similar adhesive is used) to seal the balloon to the metal tube. In the example shown, heat shrinks 9 and 10 are used to cover the balloon on both the proximal and distal ends of the balloon over the manipulator tube 5. Any commercially available heat shrink for insulating a tube can be used. Insultab's HS-714 (acrylated polyolefin) heat shrink can preferably be used. The heat shrink can be colored to facilitate visibility of the laser graduation markings. The heat shrink material sticks to itself and metal manipulator tube 5 with or without silicone glue. The inventors discovered that a heat shrink 9 has many manufacturing and reliability advantages over the conventional use of adhesive. For example, the heat shrink better retains its properties through the sterilization process as compared to adhesives. As adhesive degrades and weakens, the balloon may slide, causing patient exposure to the adhesive, and also may lead to malfunction of the balloon itself.

The heat shrink 9 seals the intrauterine balloon 7 at a first proximal position and heat shrink 10 seals the intrauterine balloon 7 at a second distal position along the most distal portion of the manipulator tube 5 forming a pair of collars around the balloon 7. The heat shrinks 9 and 10 can also be used to cover the entirety of the manipulator tube 5 at the position of collars for insulation purposes when used in conjunction with electrosurgical accessories. A portion of the balloon 7 is not covered with a heat shrink so that the balloon 7 may inflate. The uncovered portion of the balloon 7 can be adjacent a substantially enclosed cavity between the manipulator tube 5 and the balloon 7. The lumen 15 provides a pathway through the manipulator tube 5 to transport gas to the balloon 7. The lumen 15 provides a closed passage to the balloon 7 to facilitate inflation without introducing dye from the dye injection port cap 13 into the balloon 7.

Cap 8 is positioned at the most distal portion of the manipulator tube 5 and is configured to provide atraumatic insertion. The cap 8 can be approximately T-shaped, with a rounded portion and a stem portion. The stem portion of the cap 8 is positioned within the manipulator tube 5. The rounded portion extends across the diameter of the most distal portion of the manipulator tube 5. Specifically, the diameter of the rounded portion of the cap 8 extends beyond the diameter of the manipulator tube 5 and out to a similar diameter of the heat shrink 10.

The cap 8 additionally can be closed/sealed, but can alternatively include a molded channel for the passage of dye through both the rounded and stem portions of the cap 8. The channel extends entirely through the cap 8 to facilitate the passage of dye therethrough. Dye can be passed from a dye injection port cap 13 through the manipulator tube 5 and the cap 8 into the uterine cavity. Conventional caps are closed and utilize a slit in the balloon to create a passage for dye. The slit in the balloon can be open during manufacturing and closed via a seal before use of the balloon. In some instances, the seal does not work and the slit remains open, which renders the balloon inoperable. In other instances, the closed cap causes adhesive to bunch up at the tip of the manipulator tube 5. However, the balloon 7 is entirely separate from the manipulator tube 5. In particular, dye can flow through the manipulator tube 5 and through channel in the cap 8, while gas is configured to be separately passed through a lumen 15 to expand the balloon 7. Alternative examples of cap 8 include various structural configurations and numbers of molded channels passing therethrough. For example, one or more channels can pass through the center of cap 8, on either side of cap 8, and can include bifurcations into additional multiple channels (which can assist with strategically distributing dye to certain anatomy, such as to and through the fallopian tubes).

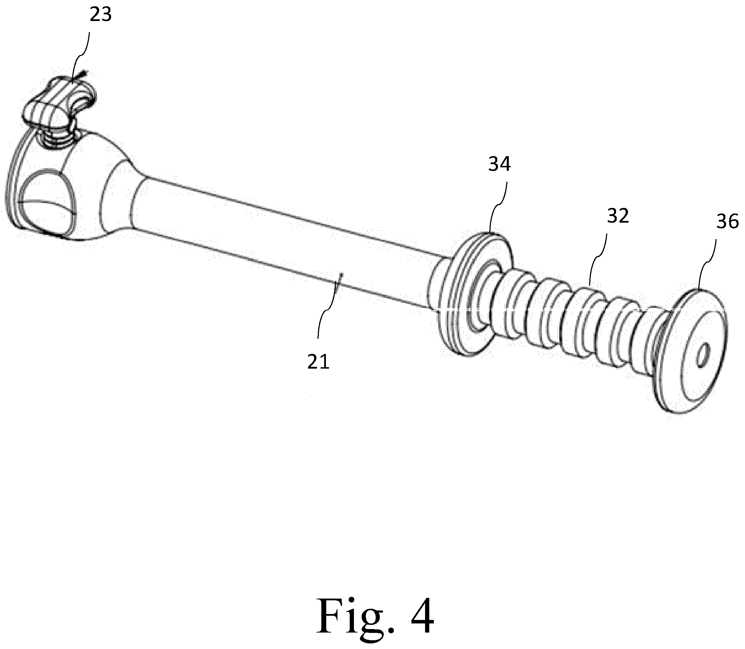

The use of the uterine manipulator devices described herein are similar to the use of the uterine manipulator device described in US Pub. App. No. 20170354436 (see, e.g., Fig. 4, and para. [0028]), as should be understood by a person of ordinary skill in the art in conjunction with a review of this disclosure.

To maintain pneumoperitoneum during use, the balloon occluder assembly 20 contains an inflatable balloon occluder 22 for vaginal occlusion by conforming to patient anatomy. The balloon occluder assembly 20 is configured to be detached from the uterine manipulator 1 and be used independently. Referring now to FIG 2A and FIG 2B, there are shown side perspective views of the balloon occluder assembly 20 in the uninflated state. Following colpotomy or similar instance and subsequent removal of the device 100, the balloon occluder assembly 20 can be removed from the uterine manipulator 1 as described below. The balloon occluder 22 of the independent balloon occluder assembly 20 can then be reinserted into the vaginal canal to act as an occluder without the uterine manipulator 1.

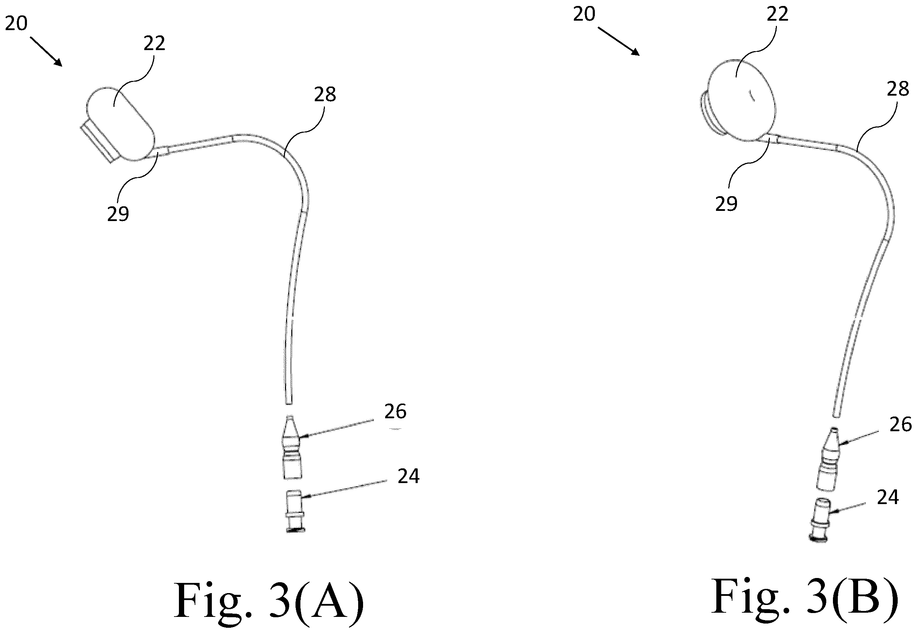

Once the balloon occluder assembly 20 is in place, balloon occluder 22 can be placed into an inflated state using check valve 24 and lumen 28. FIGS 3A and 3B show the balloon occluder assembly 20 with balloon occluder 22 in an inflated state. The lumen 28 provides a pathway to transport air to the balloon occluder 22 to facilitate inflation. The pilot balloon 26 fills with air and becomes less flexible after the balloon occluder 22 is full as an indicator of inflation. The independent balloon occluder assembly 20 can be particularly useful for example during suturing of the vaginal cuff following hysterectomy.

Referring now to FIG 4, the stem 21 comprises the thumbscrew 23 on its proximal end and a set of ribs 32 on its distal end. Balloon occluder 22 is configured to be placed over the ribs 32 on the distal portion of the stem 21. The ribs 32 can be of similar or varying sizes. In the example shown there are five ribs 32 but there can be any number of ribs 32. Alternatively, the distal end of the stem 21 can be smooth or the ribs 32 can be accomplished by other means such as but not limited to bumps, ridges, or texture. Ribs 32 help to hold and secure balloon occluder 22. In the example shown, the ribs 32 are positioned in between a proximal lip 32 and distal lip 36 which can also help to hold the balloon occluder 22 in place and prevent movement of balloon occluder 22 along the canulated tube 5.

To place the balloon occluder 20 on the stem 21 a user can slide the distal end 6 of the uterine manipulator device 1 through the opening (or lumen 22-1) of the balloon occluder 22. Balloon occluder 22 can be slid down the canulated tube 5 and stretched over the cervical cup 14 and the distal lip 36 of the stem 21. The balloon occluder 22 can sit and be held in place between the proximal and distal lips 34, 36 on the ribs 32. To remove the balloon occluder 20 from the uterine manipulator device 1 the balloon occluder 22 can be slid over the distal lip 36 and cervical cup 14 and off the distal end 6 of the canulated tube 5. Stem 21 can remain on the uterine manipulator 1 after the balloon occluder assembly 20 is removed.

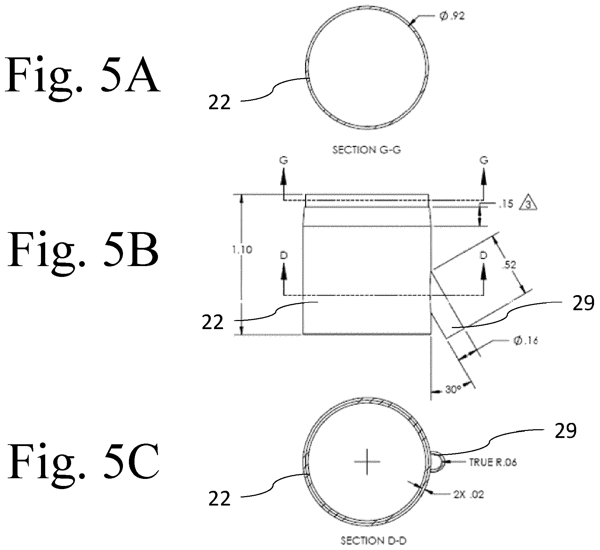

Balloon occluder 22 according to one embodiment of the invention is a torus shaped balloon with an opening configured to be slid over parts of the uterine manipulator 1. Referring now to FIG 5A-C, the balloon occluder 22 is composed of a flexible material such as silicone. Alternatively, according to an example not forming part of the invention, the balloon occluder 22 can be a urethane-based balloon or TPE for example a TPE balloon based on a styrene block copolymer, SEBS compound. As shown and according to an embodiment of the invention, the balloon occluder 22 is designed to be constructed from a single silicone molded component, which is folded over on itself and sealed circumferentially. Referring now to FIG 5B, the section G-G of the balloon occluder 22 is above where the seal is and is only a single layer of material. Section D-D shows the two layers of the balloon occluder 22 which forms the pocket for air. This pocket within the layers can be inflated through the communicatively coupled check valve 24, pilot balloon 26, and lumen 28. In the embodiment shown the balloon occluder 22 also comprises a lumen port 29 which can be formed into the body of the balloon 22 and attached to lumen 28 by a method such as overmolding. The balloon occluder 22 can be configured in other arrangements as should be known in the art. Balloon occluder 22 can be made in a variety of shapes and sizes and inflated to any desired size.

## Claims

1. A balloon occluder assembly (20), comprising:
a torus shaped balloon occluder (22) configured to be changed from a deflated state to an inflated state and comprising an inner wall and an outer wall; and
an inflation valve (24) communicatively coupled to the balloon occluder (22) via a gas passage lumen (28),
**characterized in that** the balloon occluder (22) is constructed from a single silicone molded component, which is folded over on itself and sealed circumferentially, so that the seal separates a first axial portion with only a single layer of material from a second axial portion with two layers which form a pocket for air.

2. The balloon occluder assembly (20) of claim 1, further comprising a pilot balloon (26) configured to indicate balloon inflation.

3. The balloon occluder assembly (20) of claim 1 or 2, the outer wall of the balloon occluder (22) further comprising a lumen port (29).

## Patentansprüche

1. Ballonokkluder-Anordnung (20), umfassend:
einen torusförmigen Ballonokkluder (22), der dazu konfiguriert ist, von einem entleerten Zustand in einen aufgeblasenen Zustand verändert zu werden und der eine Innenwand und eine Außenwand umfasst; und
ein Aufblasventil (24), das mit dem Ballonokkluder (22) über ein Gasdurchgangslumen (28) kommunikativ gekoppelt ist,
**dadurch gekennzeichnet, dass** der Ballonokkluder (22) aus einem einzelnen silikongeformten Bauteil konstruiert ist, das auf sich selbst zurückgefaltet und umfänglich versiegelt ist, so dass die Versiegelung einen ersten axialen Abschnitt mit nur einer einzelnen Materialschicht von einem zweiten axialen Abschnitt mit zwei Schichten trennt, die eine Lufttasche bilden.

2. Ballonokkluder-Anordnung (20) nach Anspruch 1, ferner umfassend einen Pilotballon (26), der dazu konfiguriert ist, die Ballonaufblasung anzuzeigen.

3. Ballonokkluder-Anordnung (20) nach Anspruch 1 oder 2, wobei die Außenwand des Ballonokkluder (22) ferner eine Lumenöffnung (29) umfasst.

## Revendications

1. Ensemble (20) d'occluseur à ballonnet, comprenant :
un occluseur (22) à ballonnet en forme de tore configuré pour être changé d'un état dégonflé à un état gonflé et comprenant une paroi intérieure et une paroi extérieure ; et
une valve de gonflage (24) couplée en communication à l'occluseur (22) à ballonnet par l'intermédiaire d'une lumière (28) de passage de gaz,
**caractérisé en ce que** l'occluseur (22) à ballonnet est construit à partir d'un composant moulé unique en silicone, qui est replié sur lui-même et scellé circonférentiellement, de telle sorte que le joint d'étanchéité sépare une première partie axiale avec seulement une couche unique de matériau d'une deuxième partie axiale avec deux couches qui forment une poche pour air.

2. Ensemble (20) d'occluseur à ballonnet selon la revendication 1, comprenant en outre un ballonnet pilote (26) configuré pour indiquer un gonflage de ballonnet.

3. Ensemble (20) d'occluseur à ballonnet selon la revendication 1 ou 2, la paroi extérieure de l'occluseur (22) à ballonnet comprenant en outre un orifice (29) de lumière.
